# FASCICULE DE BREVET EUROPEEN

(11) **EP 0 617 954 B1**
(45) Date de publication et mention de la délivrance du brevet: **07.05.1997**
(21) Numéro de dépôt: 94400616.2
(22) Date de dépôt: 23.03.1994
(51) Int. Cl.: A61K 7/06, A61K 7/48, A61K 7/50, A61K 7/08

(54) **Composition cosmétique contenant au moins un agent tensio-actif non-ionique du type alkylpolyglycoside et/ou polyglycérolé et au moins un copolymère réticulé d'anhydride maléique/alkyl(C1-C5) vinyléther**
Alkylpolyglycosid- und/oder Polyglycerol als nichtionisches Tensid und vernetztes Maleinsäureanhydrid/Alkyl(C1-C5)-vinylether-Copolymer enthaltende kosmetische Zusammensetzung
Cosmetic composition containing an alkylpolyglycoside and/or polyglycerol type nonionic surfactant and a maleic anhydride/alkyl(C1-C5) vinylether crosslinked copolymer

(30) Priorité: 24.03.1993 FR 9303403
(43) Date de publication de la demande: 05.10.1994
(73) Titulaire: L'OREAL, 75008 Paris (FR)
(72) Inventeur: Cauwet, Danièle, F-75011 Paris (FR); Dubief, Claude, F-78150 Le Chesnay (FR)
(74) Mandataire: Casalonga, Axel

(56) Documents cités:
- WO-A-92/08439
- GB-A- 2 139 243
- US-A- 3 666 671
- US-A- 5 034 220
- RESEARCH DISCLOSURE, no.3343, Novembre 1992, EMSWORTH, GB 'PVM/MA Decadiene Crosspolymer: A New Thickener / Stabilizer'

## Description

L'invention concerne des compositions cosmétiques contenant au moins un agent tensio-actif non-ionique de type alkylpolyglycoside et/ou polyglycérolé et au moins un copolymère réticulé d'anhydride maléique/alkyl(C₁-C₅)vinyléther.

Les gels de copolymères réticulés d'anhydride maléique/alkyl (C₁-C₅)vinyléther, hydrolysés et neutralisés, sont connus comme agents épaississants ou agents stabilisants dans des compositions cosmétiques aqueuses ou anhydres pour le soin ou le traitement des cheveux ou de la peau. Ils sont décrits dans les brevets USP-5.032.391, 5.034.220 et 5.024.779 de la Société GAF.

Les agents tensio-actifs de la famille des alkylpolyglycosides ou polyglycérolés ont déjà été préconisés dans des compositions lavantes pour les cheveux ou la peau. Ce sont des détergents doux, bien tolérés et biodégradables.

Les cheveux, agressés par les agents atmosphériques tels que la lumière ou les traitements chimiques, et lavés avec des bases lavantes classiques, sont difficilement démêlables et cet inconvénient se trouve encore accentué dans le cas de cheveux fins.

Les compositions de shampooing ne contenant que des tensioactifs non-ioniques ne conduisent pas à de bonnes propriétés cosmétiques, en particulier le démêlage des cheveux mouillés est difficile et les cheveux sont rêches.

Des compositions de lavage et moussantes contenant des agents tensio-actifs et des polymères anioniques et destinées au traitement des fibres kératiniques ont également été décrites dans la demande de brevet GB 2.139.243, les effets obtenus par cette combinaison étant une amélioration de la douceur de la mousse et de l'activité détergeante de l'agent tensio-actif.

La demanderesse vient de découvrir, de manière surprenante, que l'association dans des compositions lavantes et/ou traitantes pour matières kératiniques de copolymères réticulés d'anhydride maléque/alkyl(C₁-C₅)vinyléther à des tensio-actifs non-ioniques particuliers du type alkylpolyglycoside et/ou du type polyglycérolé, conférait à ces compositions des propriétés de démêlage considérablement améliorées. Par ailleurs, l'association conforme à l'invention permet d'obtenir une mousse très douce par rapport à la mousse généralement rêche résultant de l'utilisation d'agents non-ioniques.

En outre, la demanderesse a constaté que les compositions contenant une telle association avaient de bonnes propriétés cosmétiques telles que la douceur, un toucher agréable.

La présente invention a donc pour objet des compositions cosmétiques contenant au moins un agent tensio-actif non-ionique de type alkylpolyglycoside et/ou polyglycérolé et au moins un copolymère réticulé d'anhydride maléique/alkyl(C₁-C₅)vinyléther.

Un autre objet de l'invention est constitué par l'utilisation de ces compositions pour le traitement et/ou le lavage des matières kératiniques telles que les cheveux ou la peau.

Un autre objet de l'invention concerne un procédé de traitement cosmétique des cheveux, ou de la peau, au moyen des compositions conformes à l'invention; le procédé de lavage et de traitement des cheveux étant particulièrement préféré.

D'autres objets de l'invention apparaîtront à la lecture de la description et des exemples qui suivent.

Les compositions cosmétiques selon l'invention contiennent dans un milieu cosmétiquement acceptable, au moins un agent tensio-actif non-ionique de type alkylpolyglycoside et/ou polyglycérolé et au moins un copolymère réticulé d'anhydride maléique/alkyl(C₁-C₅)vinyléther. De façon préférentielle, la viscosité d'une solution aqueuse contenant de 0,5 à 1,5% en poids dudit copolymère hydrolysé et neutralisé, à un pH compris entre 4 et 11, mesurée à 25°C avec un viscosimètre BROOKFIELD RTV, broche du type TE à 10 tours/minute, est comprise entre 35.000 et 180.000 mPa·s (cps).

Les copolymères réticulés conformes à l'invention sont préparés par polymérisation d'anhydride maléique, d'un alkylvinyléther, d'un agent réticulant, en présence d'un initiateur de radicaux libres dans un solvant approprié. On utilise de préférence un mélange d'acétate d'éthyle et de cyclohexane (35-55% en poids/45-65% en poids) à titre de solvant.

Les agents réticulants sont utilisés de préférence dans des proportions comprises entre 1 et 5% en mole par rapport au monoalkylvinyléther. Ils sont, par exemple, choisis parmi les composés insaturés tels que les divinyléthers de diol aliphatique; les divinyléthers de polyéthylèneglycol; le 1,7-octadiène; le 1,9-décadiène; le divinylbenzène; le N,N'-bis-méthylèneacrylamide; le diacrylate de polyéthylèneglycol; le triacrylate de triméthylolpropane; le diacrylate de propylèneglycol; les mono- ou diacrylates de polyols; la triallylamine; la tétraallyléthylènediamine et le diallylphtalate.

On utilise plus particulièrement le 1,7-octadiène ou le 1,9-décadiène.

Les initiateurs de radicaux libres sont utilisés de préférence dans des proportions comprises entre 0,001 et 1% en poids par rapport à la composition de monomères. Ils sont notamment choisis parmi l'azo-bis-isobutyronitrile, le peroxyde de benzoyle, le peroxyde de lauroyle, le peroxyde de caprylyle, le peroxyde d'acétyle, le peroxyde d'acétyl-benzoyle, le peroxyde de ditertiobutyle, l'azo-bis-(2,4-diméthylvaléronitrile).

La polymérisation est effectuée à une température comprise entre 0 et 150°C, de préférence entre 50 et 100°C et plus particulièrement entre 60 et 80°C.

Les copolymères réticulés ainsi obtenus sont sous forme de poudre.

Lorsqu'ils sont hydrolysés et neutralisés dans une solution aqueuse basique comprenant de 0,5 à 1,5% en poids de copolymère et une quantité suffisante d'un agent neutralisant telle qu'une solution d'hydroxyde alcalin à 10% ou d'ammoniaque à 30%, ou d'une alcanolamine comme la monoéthanolamine, la diéthanolamine, la triéthanolamine, l'aminométhylpropanol, l'aminométhylpropanediol, la viscosité mesurée avec le viscosimètre BROOKFIELD RTV, broche du type TE à 10 tours/minute, à 25°C et à un pH compris entre 4 et 11 et en particulier entre 6 et 7,4, est préférentiellement comprise entre 35.000 et 180.000 mPa·s (cps).

Les copolymères préférés utilisés selon la présente invention sont les copolymères d'anhydride maléique/méthylvinyléther, réticulés par 1 à 5% en mole par rapport au méthylvinyléther de 1,9-décadiène ou de 1,7-octadiène.

Un produit commercial de ce type est par exemple le STABILEZE 06 vendu par la Société ISP.

Les copolymères réticulés de l'invention sont présents dans les compositions cosmétiques dans des concentrations comprises entre 0,2 et 5% en poids, et de préférence entre 0,3 et 3% en poids.

Les alkylpolyglycosides utilisables conformément à l'invention répondent en particulier à la formule (II) suivante:

R(C₆H₁₀O₅)ₓ-H (II)

correspondant à la structure développée (III) : 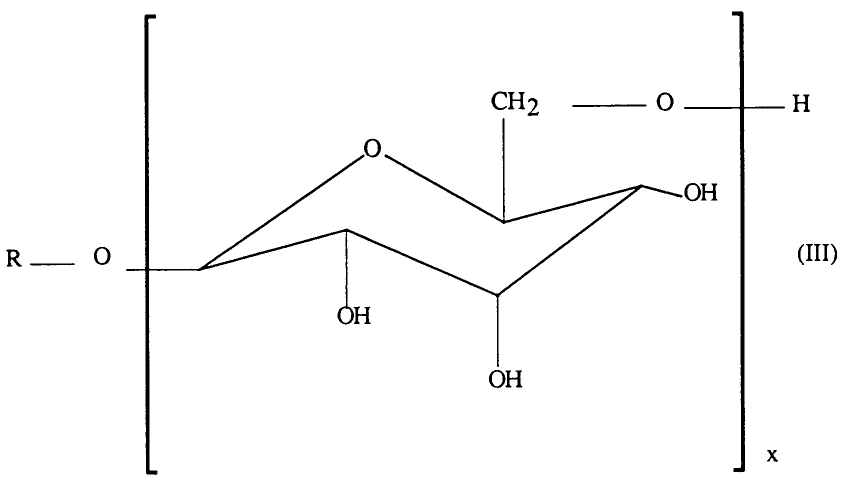 dans laquelle :
R désigne un radical ou un mélange de radicaux alkyles ou alcényles à chaîne droite ou ramifiée en C₈-C₂₄;
x est un nombre compris entre 1 et 15.

Les composés alkylpolyglycosides de formule développée (III) définie ci-dessus, utilisés conformément à l'invention, sont de préférence représentés par les produits vendus par la Société HENKEL sous la dénomination APG, tels que les produits APG 300, APG 350, APG 500, APG 550, APG 625, APG base 10-12; PLANTAREN 300, 600, 1200 CS/UP, 2000 CS/UP; les produits vendus par la Société SEPPIC sous les dénominations TRITON CG 110 (ou ORAMIX CG 110) et TRITON CG 312 (ou ORAMIX NS 10); ceux vendus par la Société BASF sous la dénomination LUTENSOL GD 70.

Les agents tensio-actifs non-ioniques du type polyglycérolé utilisés conformément à la présente invention, sont choisis de préférence parmi les composés polyhydroxypropyléthers suivants :
(A) Les composés répondant à la formule (IV) :

   RO [ C₃H₅(OH)⁆ₙH (IV)

   dans laquelle le groupement [C₃H₅ (OH)] représente les structures suivantes, prises ensemble ou séparément :

   ⁅CH₂ CH OH - CH₂ - O⁆ (IVa)

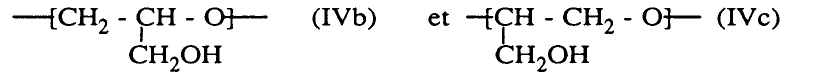

   et R et n ont une des significations ci-après :
   a) R représente un radical ou un mélange de radicaux alkyle en C₁₀-C₁₄ et n est un nombre entier ou décimal de 2 à 10 et de préférence 3 à 6.
   b) R représente un reste :

      R₂ CONH CH₂ - CH₂O CH₂ - CH₂ - (V)

      où R₂ désigne un radical ou un mélange de radicaux alkyle et/ou alcényle en C₁₁-C₁₇ et n désigne un nombre entier ou décimal de 1 à 5 et de préférence de 1,5 à 4.
   c) R représente un reste :

      R₃ - CHOH - CH₂ - (VI)

      où R₃ désigne un radical aliphatique, cycloaliphatique, arylaliphatique en C₇-C₂₁ et leurs mélanges, les chaînes aliphatiques désignant en particulier des chaînes alkyle pouvant comporter de 1 à 6 groupements éther, thioéther et/ou hydroxyméthylène et n désigne un nombre entier ou décimal de 1 à 10.

   Ces tensioactifs de formule (IV) peuvent être préparés selon les procédés décrits dans les brevets FR 1 477 048, 2 328 763 et 2 091 516;
(B) Les composés préparés par condensation, en catalyse acide, de 2 à 10 et de préférence de 2,5 à 6 moles de glycidol par mole d'alcool ou d'alpha-diol contenant 10 à 14 atomes de carbone, à une température de 50 à 120°C, le glycidol étant ajouté lentement à l'alcool ou à l'alpha-diol. Le procédé de préparation de ces composés est décrit dans le brevet FR-A-2 169 787;
(C) Les composés polyhydroxypropyléthers préparés par polyaddition de monochlorhydrine du glycérol sur un composé organique polyhydroxylé en présence d'une base forte, avec élimination au fur et à mesure de l'eau par distillation. Ces composés sont décrits dans le brevet français FR-A-2 574 786.

Parmi les tensio-actifs non ioniques de la famille des polyhydroxypropyléthers décrit dans les paragraphes (A), (B) et (C) ci-dessus, les composes préférés sont représentés par les formules:
(α) 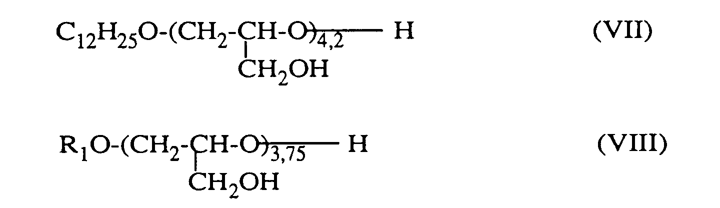 où R₁ désigne un mélange de radicaux alkyles en C₁₀H₂₁ et C₁₂ H_{25;}
(β) les composés préparés par condensation en catalyse alcaline, de 3,5 moles glycidol sur un alphadiol ayant 12 atomes de carbone, selon le procédé décrit dans le brevet FR-A-2 091 516;
(γ) les composés répondant à la formule :

   R₂-CONH-CH₂-CH₂-O-CH₂-CH₂-O-(CH₂-CHOH-CH₂-O)̵_{3,5} H (IX)

   où R₂ désigne un mélange de radicaux comprenant les radicaux alkyle et alcényle suivants :
   C₁₁H₂₃,C₁₃H₂₇, les radicaux dérivés des acides gras du coprah et le radical dérivé de l'acide oléique;
(δ) les composés préparés par condensation de 3,5 moles de glycidol sur un mélange d'alphadiols en C₁₁-C₁₄, décrits dans le brevet FR-A-2 091 516.

Le tensio-actif non ionique polyhydroxypropyléther obtenu par condensation de la monochlorhydrine du glycérol (2,5 moles) en présence de soude sur le dodécanediol-1,2, est plus particulièrement préféré.

Si les compositions selon l'invention ne sont pas utilisées pour le lavage des matières kératiniques, le ou les agents tensio-actifs non ioniques de type alkylpolyglycoside et/ou polyglycérolé sont utilisés dans de telles compositions dans des proportions comprises entre 0,1 et 5 % en poids par rapport au poids total de la composition. Ces compositions sont utilisées notamment comme compositions à rincer, appliquées avant ou après un shampooing, une coloration, une décoloration, une permanente, un défrisage, comme composition de coloration ou de permanente.

Si les compositions selon l'invention sont des compositions lavantes, elles contiennent le ou les tensio-actifs non ioniques du type alkylpolyglycoside et/ou polyglycérolé dans des proportions comprises entre 5 et 30 % en poids par rapport au poids total de la composition et plus particulièrement entre 5 et 20 % en poids.

Le pH des compositions conforme à l'invention est généralement compris entre 2 et 9 plus particulièrement entre 3 et 6.

Dans la mesure où le milieu cosmétiquement acceptable de la composition selon l'invention est un milieu aqueux, il peut être constitué uniquement par de l'eau ou par un mélange d'eau et d'un solvant cosmétiquement acceptable, tel que des alcools inférieurs en C₁-C₄, comme l'éthanol, l'isopropanol, le n-butanol; les alkylèneglycols, comme le propylèneglycol, les éthers de glycols.

Les compositions selon l'invention peuvent se présenter sous forme de liquides plus ou moins épaissis, de gels, d'émulsions (laits ou crèmes), de lotions hydroalcooliques, de dispersions, ou de mousses aérosol.

Les compositions sont par exemples des lotions, des laits ou des crèmes émollients, des lotions, des laits ou des crèmes pour les soins des matières kératiniques, des crèmes ou des laits démaquillants, des bases de fonds de teint, des lotions, des laits ou des crèmes anti-solaires, des lotions, des laits ou des crèmes de bronzage artificiel, des crèmes ou des mousses de rasage, des lotions après rasage, des masques pour le visage, des produits de maquillage pour les yeux, des fards et fonds de teint pour le visage, des vernis à ongles, des shampooings, des produits pour le bain ou la douche, des compositions à rincer, à appliquer avant ou après un shampooing, une coloration, une décoloration, une permanente ou un défrisage.

Les compositions conformes à l'invention peuvent éventuellement contenir, en outre, divers additifs qui n'altèrent pas les propriétés des compositions, telles que des agents tensioactifs anioniques, cationiques, amphotères ou zwittérioniques, des agents tensioactifs non ioniques autres que ceux décrits précédemment, des polymères anioniques, non ioniques, cationiques ou amphotères, des protéines, des huiles hydrocarbonées telles que des huiles de synthèse comme les isoparaffines ou des huiles minérales, végétales ou animales, des huiles, cires, résines et/ou gommes de silicone, des agents acidifiants ou alcalinisants, des agents conservateurs, des actifs, d'autres épaisissants, des agents de mise en suspension, des adoucissants, des filtres solaires, des parfums, des biocides, des antioxydants, des composés fluorés, des pigments ou d'autres adjuvants couramment utilisés en cosmétique.

Les compositions conformes à l'invention sont appliqués sur la peau ou les cheveux dans une quantité efficace cosmétiquement, fonction de la nature de la composition.

Une application particulière des compositions selon l'invention est l'application en tant que composition de lavage et de traitement cosmétique des matières kératiniques, de préférence de la peau et des cheveux et plus particulièrement en tant que shampooing. Dans ce cas-là, le shampooing est appliqué sur les cheveux humides ou secs dans des quantités efficaces pour les laver, cette application étant suivie d'un rinçage.

Les exemples suivants sont destinés à illustrer l'invention sans pour autant présenter un caractère limitatif.

### EXEMPLE 1

On prépare un **shampooing** de composition suivante :

| | |
|---|---|
| - Alkyl(C₉-C₁₀-C₁₁/20-40-40)polyglycoside (1,4), vendu à 50% de MA sous la dénomination "APG 300" par la Société HENKEL | 10 g MA |
| - Copolymère anhydride maléique/méthylvinyléther réticulé par du 1,9-décadiène (viscosité d'une solution à 0,5% : 110.000 mPa·s (cps)) | 1,5 g |
| - Laurylsulfate de triéthanolamine vendu à 40% de MA | 5 g MA |
| - Parfum, conservateur qs | |
| - Hydroxyde de sodium qs pH=7 | |
| - Eau | qsp 100 g |

### EXEMPLE 2

On prépare un **shampooing** de composition suivante :

| | |
|---|---|
| - Alkyl(C₉-C₁₀-C₁₁/20-40-40)polyglycoside (1,4), vendu à 50% de MA sous la dénomination "APG 300" par la Société HENKEL | 10 g MA |
| - Copolymère anhydride maléique/méthylvinyléther réticulé par du 1,9-décadiène (viscosité d'une solution à 0,5% : 50.000 mPa·s (cps)) | 1,5 g |
| - Perhydrosqualène | 2 g |
| - Parfum, conservateur qs | |
| - Hydroxyde de sodium qs pH=7 | |
| - Eau | qsp 100 g |

### EXEMPLE 3

On prépare un **après-shampooing** à rincer de composition suivante :

| | |
|---|---|
| - Alkyl(C₉-C₁₀-C₁₁/20-40-40)polyglycoside (1,4), vendu à 50% de MA sous la dénomination "APG 300" par la Société HENKEL | 2 g MA |
| - Copolymère anhydride maléique/méthylvinyléther réticulé par du 1,9-décadiène, vendu sous la dénomination "STABILEZE 06" par la Société ISP | 1 g |
| - Heptaméthylnonane vendu sous la dénomination "ARLAMOL HD" par la Société ICI | 5 g |
| - Conservateur, parfum qs | |
| - Hydroxyde de sodium qs pH=6 | |
| - Eau | qsp 100 g |

### EXEMPLE 4

On prépare un **après-shampooing** à rincer de composition suivante :

| | |
|---|---|
| - Alkyl(C₉-C₁₀-C₁₁/20-40-40)polyglycoside (1,4), vendu à 50% de MA sous la dénomination "APG 300" par la Société HENKEL | 2 g MA |
| - Copolymère anhydride maléique/méthylvinyléther réticulé par du 1,9-décadiène (viscosité d'une solution à 0,5% : 110.000 mPa·s (cps)) | 1,75 g |
| - Hydrolysat de protéine de soie quaternisée par du chlorure de cocoyl diméthyl ammonium, vendu en solution aqueuse à 32% de MA sous la dénomination "CROSILQUAT" par la Société CRODA | 3 g MA |
| - Conservateur, parfum qs | |
| - Hydroxyde d sodium qs pH=7 | |
| - Eau | qsp 100 g |

### EXEMPLE 5

On prépare un **shampooing** de composition suivante :

| | |
|---|---|
| - Tensio-actif non-ionique poly(hydroxypropyléther) préparé par condensation, en catalyse alcaline, de 3,5 moles de glycidol sur un mélange d'alphadiols ayant 10 à 14 atomes de carbone, selon le procédé décrit dans le brevet français n° 2.091.516 | 20 g |
| - Copolymère anhydride maléique/méthylvinyléther réticulé par du 1,9-décadiène (viscosité d'une solution à 0,5% : 110.000 mPa·s (cps)) | 1 g |
| - Perhydrosqualène | 2 g |
| - Conservateur qs | |
| - Hydroxyde de sodium qs pH=5 | |
| - Eau | qsp 100 g |

### EXEMPLE 6

On prépare un **shampooing** de composition suivante :

| | |
|---|---|
| - Alkyl(C₉-C₁₀-C₁₁/20-40-40)polyglycoside (1,4), vendu à 50% de MA sous la dénomination "APG 300" par la Société HENKEL | 15 g MA |
| - Copolymère anhydride maléique/méthylvinyléther réticulé par du 1,9-décadiène (viscosité d'une solution à 0,5% : 50.000 mPa·s (cps)) | 1,5 g |
| - Polydiméthyldiphénylsiloxane vendu sous la dénominantion "SILBIONE HUILE 70641 V 200" par la Société RHONE POULENC | 3 g |
| - Conservateur qs | |
| - Hydroxyde de sodium qs pH=6 | |
| - Eau | qsp 100 g |

### EXEMPLE 7

On prépare un **après-shampooing** à rincer de composition suivante :

| | |
|---|---|
| - Alkyl(C₁₂-C₁₄-C₁₆/68-26-6)polyglycoside (1,4), vendu à 50% de MA sous la dénomination "APG 600" par la Société HENKEL | 0,2 g MA |
| - Copolymère anhydride maléique/méthylvinyléther réticulé par du 1,9-décadiène (viscosité d'une solution à 0,5% : 110.000 mPa·s (cps)) | 5 g |
| - Huile de colza | 3 g |
| - Conservateur qs | |
| - Hydroxyde de sodium qs pH=4 | |
| - Eau | qsp 100 g |

### EXEMPLE 8

On prépare une **lotion** de mise en plis de composition suivante :

| | |
|---|---|
| - Alkyl(C₉-C₁₀-C₁₁/20-40-40)polyglycoside (1,4), vendu à 50% de MA sous la dénomination "APG 300" par la Société HENKEL | 0,1 g MA |
| - Copolymère anhydride maléique/méthylvinyléther réticulé par du 1,9-décadène, vendu sous la dénomination "STABILEZE 06" par la Société ISP | 1 g |
| - Copolymère vinylpyrrolidone/acétate de vinyle en solution alcoolique à 50% de MA (PVP/VA E 735 de ISP) | 1 g |
| - Amino-2 méthyl-2 propanol qs pH=7,5 | |
| - Eau | qsp 100 g |

### EXEMPLE 9

On prépare un **gel douche** de composition suivante :

| | |
|---|---|
| - APG 300 | 15 g MA |
| - STABILEZE 06 | 2,5 g |
| - Lauryl éther sulfate de sodium à 2,2 moles d'oxyde d'éthylène en solution aqueuse à 28% de MA | 8 g MA |
| - Copolymère acide acrylique/chlorure de diméthyl diallyl ammonium (20/80) (Merquat 280 de Merck) | 0,5 g MA |
| - Conservateur, parfum qs | |
| - NaOH qs pH=6,5 | |
| - Eau | qsp 100 g |

## Revendications

1. Composition cosmétique, caractérisée en ce qu'elle contient dans un milieu cosmétiquement acceptable, au moins un agent tensio-actif non-ionique de la famille des alkylpolyglycosides et/ou un agent tensio-actif non-ionique polyglycérolé et au moins un copolymère réticulé d'anhydride maléique/alkyl(C₁-C₅)vinyléther.

2. Composition selon la revendication 1, caractérisée par le fait que le copolymère réticulé d'anhydride maléique/alkyl(C₁-C₅)vinyléther en solution dans l'eau à une concentration de 0,5 à 1,5%, hydroxylé et neutralisé à un pH compris entre 4 et 11, a une viscosité comprise entre 35.000 et 180.000 mPa·s (cps), mesurée à 25°C avec un viscosimètre BROOKFIELD RTV, broche du type TE à 10 tours/minute.

3. Composition selon les revendications 1 ou 2, caractérisée par le fait qu'elle contient un copolymère d'anhydride maléique/alkylvinyléther réticulé par 1 à 5% en mole par rapport au monoalkylvinyléther d'un agent réticulant choisi parmi les divinyléthers de diol aliphatique; les divinyléthers de polyéthylèneglycol; le 1,7-octadiène; le 1,9-décadiène; le divinylbenzène; le N,N'-bis-méthylèneacrylamide; le diacrylate de polyéthylèneglycol; le triacrylate de triméthylolpropane; le diacrylate de propylèneglycol; les mono- ou diacrylates de polyols; la triallylamine; la tétraallyléthylènediamine; le diallylphtalate.

4. Composition selon les revendications 1 à 3, caractérisée par le fait qu'elle contient un copolymère d'anhydride maléique/méthylvinyléther réticulé par 1 à 5% en mole par rapport au méthylvinyléther de 1,9-décadiène ou de 1,7-octadiène.

5. Composition selon l'une quelconque des revendications 1 à 4, caractérisée en ce que les agents tensio-actifs non-ioniques de la famille des alkylpolyglycosides répondent à la formule suivante :
R(C₆H₁₀O₅)ₓ-H (II)
correspondant à la structure développée suivante : 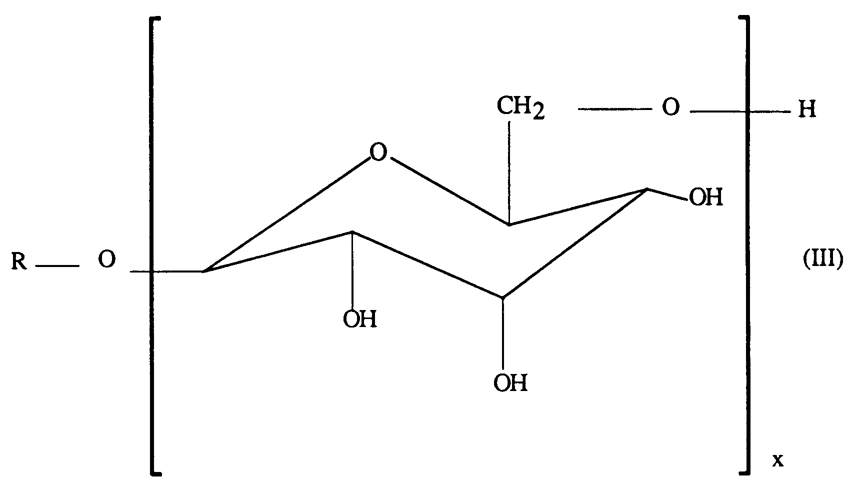 dans laquelle :
R désigne un radical ou un mélange de radicaux alkyle ou alcényle à chaîne droite ou ramifiée en C₈-C₂₄ ;
x désigne un nombre compris entre 1 et 15.

6. Composition selon l'une des revendications 1 à 5, caractérisée en ce que les agents tensioactifs non-ioniques polyglycérolés sont choisis parmi les polyhydroxypropyléthers suivants :
(A) Les composés repondant à la formule (IV) :
RO [ C₃H₅ (OH)⁆ₙH (IV)
dans laquelle le groupement [C₃-H₅ (OH)] représente les structures suivantes, prises ensemble ou séparément :
⁅CH₂CHOH - CH₂ - O⁆ (IVa)
, et 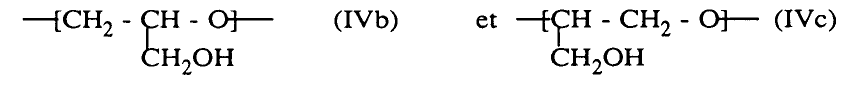 et R et n ont une des significations ci-après :
a) R représente un radical ou un mélange de radicaux alkyle en C₁₀-C₁₄ et n est un nombre entier ou décimal de 2 à 10 et de préférence de 3 à 6.
b) R représente un reste :
R₂ CONH CH₂ - CH₂OCH₂ - CH₂ - (V)
où R₂ désigne un radical ou un mélange de radicaux alkyle et/ou alcényle en C₁₁-C₁₇ et n désigne un nombre entier ou décimal de 1 à 5 et de préférence de 1,5 à 4.
c) R représente un reste :
R₃ - CHOH - CH₂ - (VI)
où R₃ désigne un radical aliphatique, cycloaliphatique, arylaliphatique en C₇-C₂₁ et leurs mélanges, les chaînes aliphatiques désignant en particulier des chaînes alkyle pouvant comporter de 1 à 6 groupements éther, thioéther et/ou hydroxyméthylène et n désigne un nombre entier ou décimal de 1 à 10.
(B) Les composés préparés par condensation, en catalyse acide, de 2 à 10 et de préférence de 2,5 à 6 moles de glycidol par mole d'alcool ou d'alpha-diol contenant 10 à 14 atomes de carbone, à une température de 50 à 120°C, le glycidol étant ajouté lentement à l'alcool ou à l'alpha-diol.
(C) Les composés polyhydroxypropyléthers préparés par polyaddition de monochlorhydrine du glycérol sur un composé organique polyhydroxylé en présence d'une base forte, avec élimination au fur et à mesure de l'eau par distillation.

7. Composition selon la revendication 6, caractérisée en ce que les agents tensioactifs non-ioniques polyglycérolés sont choisis parmi les polyhydroxypropyléthers suivants :
(α) 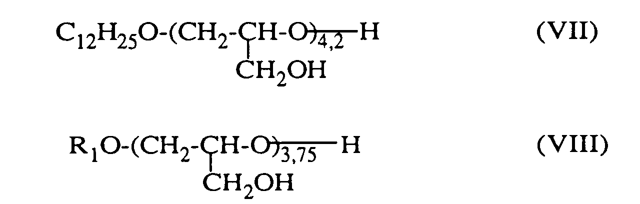 où R₁ désigne un mélange de radicaux alkyles en C₁₀H₂₁ et C₁₂ H₂₅;
(β) les composés préparés par condensation en catalyse alcaline, de 3,5 moles de glycidol sur un alphadiol ayant 12 atomes de carbone.
(γ) les composés répondant à la formule :
R₂-CONH-CH₂-CH₂-O-CH₂-CH₂-O-(CH₂-CHOH-CH₂-O)̵_{3,5}H (IX)
où R₂ désigne un mélange de radicaux comprenant les radicaux alkyle et alcényle suivants :
C₁₁H₂₃, C₁₃H₂₇, les radicaux dérivés des acides gras du coprah et le radical dérivé de l'acide oléique;
(δ) les composés préparés par condensation de 3,5 moles de glycidol sur un mélange d'alphadiols en C₁₁-C₁₄ ;

8. Composition selon l'une quelconque des revendications 1 à 7, caractérisée en ce qu'elle renferme entre 0,2 et 5 % en poids et de préférence entre 0,3 et 3 % en poids par rapport au poids total de la composition, de copolymère réticulé.

9. Composition selon l'une quelconque des revendications 1 à 8, caractérisée en ce qu'elle renferme 0,1 à 5 % en poids par rapport au poids total de la composition, d'agents tensio-actifs non-ioniques de la famille des alkylpolyglycosides et/ou polyglycérolés.

10. Composition selon l'une quelconque des revendications 1 à 8, caractérisée en ce qu'elle renferme 5 à 30 % en poids, de préférence 5 à 20 % en poids par rapport au poids total de la composition, d'agents tensio-actifs non-ioniques de la famille des alkylpolyglycosides et/ou polyglycérolés.

11. Composition selon l'une quelconque des revendications 1 à 10, caractérisée en ce que le milieu cosmétiquement acceptable est un milieu aqueux constitué uniquement par de l'eau ou par un mélange d'eau et d'un solvant cosmétiquement acceptable.

12. Composition selon l'une quelconque des revendications 1 à 11, caractérisée en ce qu'elle se présente sous forme de liquide plus ou moins épaissi, de gel, d'émulsion, de lotion hydroalcoolique, de dispersion, ou de mousse aérosol.

13. Composition selon l'une quelconque des revendications 1 à 12, caractérisée en ce qu'elle contient des agents tensio-actifs anioniques, cationiques, amphotères ou zwitterioniques, d'autres agents tensioactifs non-ioniques, des polymères anioniques, cationiques, non-ioniques ou amphotères, des protéines, des huiles hydrocarbonées, des huiles, cires, résines ou gommes de silicone, des agents acidifiants ou alcalinisants, des agents conservateurs, des actifs, d'autres épaississants, des agents de mise en suspension, des adoucissants, des filtres solaires, des parfums, des biocides, des antioxydants, des composés fluorés, des pigments ou d'autres adjuvants couramment utilisés en cosmétique.

14. Utilisation cosmétique de la composition telle que définie dans l'une quelconque des revendications 1 à 13, pour le traitement et/ou le lavage de matières kératiniques constituées par les cheveux ou la peau.

15. Procédé de traitement cosmétique, caractérisé en ce qu'il consiste à appliquer sur la peau ou les cheveux, une quantité cosmétiquement efficace d'une composition selon l'une quelconque des revendications 1 à 13 .

16. Procédé de traitement cosmétique selon la revendication 15, caractérisé en ce que la composition est appliquée en une quantité cosmétiquement efficace pour les laver sur les cheveux humides ou secs, et qu'on procède ensuite à un rinçage à l'eau.

## Claims

1. Cosmetic composition, characterized in that it contains, in a cosmetically acceptable medium, at least one nonionic surface-active agent from the class of alkyl polyglycosides and/or one nonionic polyglycerolated surface-active agent and at least one crosslinked copolymer of maleic anhydride/(C₁-C₅)alkyl vinyl ether.

2. Composition according to Claim 1, characterized in that the crosslinked copolymer of maleic anhydride/(C₁-C₅)alkyl vinyl ether dissolved in water at a concentration of 0.5 to 1.5 %, which copolymer is hydrolysed and neutralized at a pH between 4 and 11, has a viscosity of between 35,000 and 180,000 mPa.s (cps), measured at 25°C with a BROOKFIELD RTV viscometer, with a spindle of the type TE at 10 revolutions/minute.

3. Composition according to Claim 1 or 2, characterized in that it contains a maleic anhydride/alkyl vinyl ether copolymer crosslinked with 1 to 5 mol %, relative to the monoalkyl vinyl ether, of a crosslinking agent chosen from aliphatic diol divinyl ethers; polyethylene glycol divinyl ethers; 1,7-octadiene; 1,9-decadiene; divinylbenzene; N,N'-methylenebisacrylamide; polyethylene glycol diacrylate; trimethylolpropane triacrylate; propylene glycol diacrylate; polyol mono- or diacrylates; triallylamine; tetraallylethylenediamine; diallyl phthalate.

4. Composition according to Claims 1 to 3, characterized in that it contains a maleic anhydride/methyl vinyl ether copolymer crosslinked with 1 to 5 mol %, relative to the methyl vinyl ether, of 1,9-decadiene or of 1,7-octadiene.

5. Composition according to any one of Claims 1 to 4, characterized in that the nonionic surface-active agents of the alkyl polyglycoside class correspond to the following formula:
R(C₆H₁₀O₅)ₓ-H (II)
corresponding to the following structural formula: 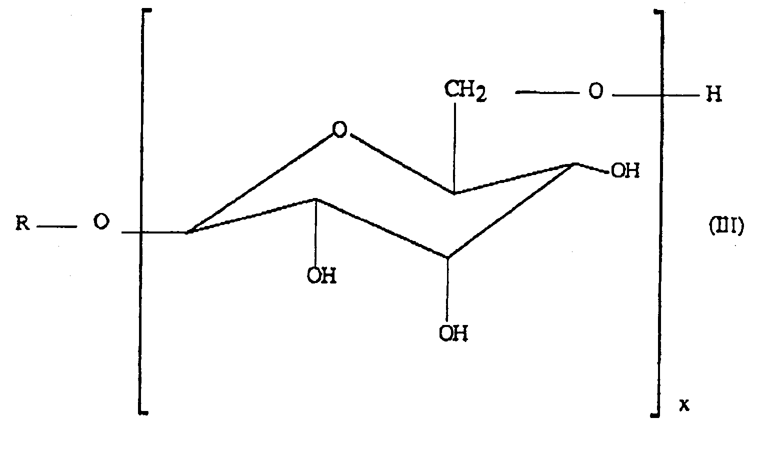 in which:
R denotes one or a mixture of alkyl or alkenyl radicals having a straight or branched C₈-C₂₄ chain;
x denotes a number between 1 and 15.

6. Composition according to one of Claims 1 to 5, characterized in that the polyglycerolated nonionic surface-active agents are chosen from the following polyhydroxypropyl ethers:
(A) The compounds corresponding to the formula (IV):
RO [ C₃H₅(OH)⁆ₙH (IV)
in which the group [C₃H₅(OH)] represents the following structures, taken together or separately:
⁅CH₂CHOH-CH₂-O⁆ (IVa),
and 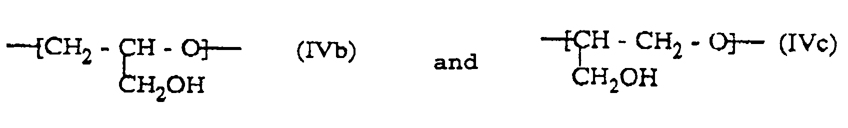 and R and n have one of the meanings below:
a) R represents one or a mixture of C₁₀-C₁₄ alkyl radicals and n denotes a whole or decimal number from 2 to 10, and preferably 3 to 6.
b) R represents a residue:
R₂CONHCH₂-CH₂OCH₂-CH₂- (V)
where R₂ denotes one or a mixture of C₁₁-C₁₇ alkyl and/or alkenyl radicals and n denotes a whole or decimal number from 1 to 5, and preferably from 1.5 to 4.
c) R represents a residue:
R₃-CHOH-CH₂- (VI)
where R₃ denotes a C₇-C₂₁ aliphatic, cycloaliphatic or arylaliphatic radical and their mixtures, the aliphatic chains in particular denoting alkyl chains which may contain from 1 to 6 ether, thioether and/or hydroxymethylene groups and n denotes a whole or decimal number from 1 to 10.
(B) The compounds prepared by acid-catalysed condensation of 2 to 10, and preferably of 2.5 to 6, moles of glycidol per mole of alcohol or of alpha-diol containing 10 to 14 carbon atoms, at a temperature of 50 to 120°C, glycidol being added slowly to the alcohol or to the alpha-diol.
(C) The polyhydroxypropyl ether compounds prepared by polyaddition of glycerol monochlorohydrin with a polyhydroxylated organic compound in the presence of a strong base, with progressive removal of the water by distillation.

7. Composition according to Claim 6, characterized in that the polyglycerolated nonionic surface-active agents are chosen from the following polyhydroxypropyl ethers:
(α) 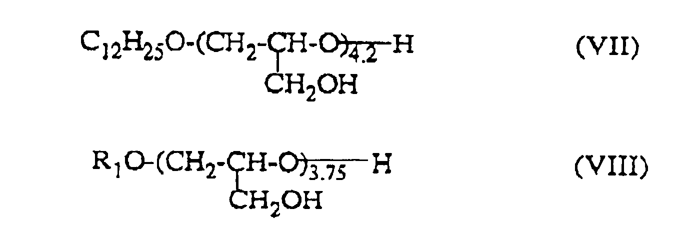 where R₁ denotes a mixture of C₁₀H₂₁ and C₁₂H₂₅ alkyl radicals;
(β) the compounds prepared by base-catalysed condensation of 3.5 moles of glycidol with an alpha-diol containing 12 carbon atoms.
(γ) the compounds corresponding to the formula:
R₂-CONH-CH₂-CH₂-O-CH₂-CH₂-O-(CH₂-CHOH-CH₂-O)̵_{3.5}H (IX)
where R₂ denotes a mixture of radicals comprising the following alkyl and alkenyl radicals:
C₁₁H₂₃, C₁₃H₂₇, radicals derived from coconut fatty acids and the radical derived from oleic acid;
(δ) the compounds prepared by condensation of 3.5 moles of glycidol with a mixture of C₁₁-C₁₄ alpha-diols.

8. Composition according to any one of Claims 1 to 7, characterized in that it contains between 0.2 and 5 % by weight, and preferably between 0.3 and 3 % by weight, relative to the total weight of the composition, of crosslinked copolymer.

9. Composition according to any one of Claims 1 to 8, characterized in that it contains 0.1 to 5 % by weight, relative to the total weight of the composition, of nonionic surface-active agents from the alkyl polyglycoside and/or polyglycerolated class.

10. Composition according to any one of Claims 1 to 8, characterized in that it contains 5 to 30 % by weight, preferably 5 to 20 % by weight, relative to the total weight of the composition, of nonionic surface-active agents from the alkyl polyglycoside and/or polyglycerolated class.

11. Composition according to any one of Claims 1 to 10, characterized in that the cosmetically acceptable medium is an aqueous medium which consists exclusively of water or of a mixture of water and a cosmetically acceptable solvent.

12. Composition according to any one of Claims 1 to 11, characterized in that it takes the form of a more or less thickened liquid, a gel, an emulsion, an aqueous-alcoholic lotion, a dispersion or an aerosol foam.

13. Composition according to any one of Claims 1 to 12, characterized in that it contains anionic, cationic, amphoteric or zwitterionic surface-active agents, other nonionic surface-active agents, anionic, nonionic, cationic or amphoteric polymers, proteins, hydrocarbon oils, silicone oils, waxes, resins or gums, acidifying or basifying agents, preservatives, active agents, other thickeners, suspension-forming agents, softeners, sunscreen agents, perfumes, biocides, antioxidants, fluorine-containing compounds, pigments or other adjuvants commonly used in cosmetics.

14. Cosmetic use of the composition as defined in any one of Claims 1 to 13 for treating and/or washing keratinous matter consisting of hair or skin.

15. Cosmetic treatment process, characterized in that it consists in applying to the skin or to hair a cosmetically effective quantity of a composition according to any one of Claims 1 to 13.

16. Cosmetic treatment process according to Claim 15, characterized in that the composition is applied to wet or dry hair in a cosmetically effective quantity to wash it, and in that this is followed by rinsing with water.

## Patentansprüche

1. Komsetische Zusammensetzung,
dadurch **gekennzeichnet,** daß
sie in einem kosmetisch geeigneten Medium mindestens ein nicht-ionisches oberflächenaktives Mittel der Familie der Alkylpolyglycoside und/oder ein nicht-ionisches oberflächenaktives Mittel einer Polyglycerylverbindung sowie mindestens ein vernetztes Copolymer aus Maleinanhydrid/C₁₋₅-Alkylvinylether enthält.

2. Zusammensetzung gemäß Anspruch 1,
dadurch **gekennzeichnet,** daß
das vernetzte Copolymer aus Maleinanhydrid/C₁₋₅-Alkylvinylether in wässriger Lösung bei einer Konzentration von 0,5 bis 1,5%, hydrolysiert und neutralisiert, bei einem pH-Wert von 4 bis 11, eine Viskosität von 35000 bis 180000 mPa x s (cps) aufweist, gemessen bei 25°C mit einem Viskosimeter BROOKFIELD RTV, Spindel vom Typ TE mit 10 Umdrehungen/Minute.

3. Zusammensetzung gemäß Anspruch 1 oder 2,
dadurch **gekennzeichnet,** daß
sie ein Maleinanhydrid/Alkylvinylether-Copolymer enthält, das mit 1 bis 5 Mol%, bezogen auf den Monoalkylvinylether, eines Vernetzungsmittels vernetzt ist, das aus Divinylethern aliphatischer Diole, Divinylethern von Polyethylenglycol, 1,7-Octadien, 1,9-Decadien, Divinylbenzol, N,N'-Bismethylenacrylamid, Polyethylenglycoldiacrylat, Trimethylpropantriacrylat, Propylenglycoldiacrylat, Mono- oder Diacrylaten von Polyolen, Triallylamin, Tetraallylethylendiamin und aus Diallylphthalat ausgewählt ist.

4. Zusammensetzung gemäß einem der Ansprüche 1 bis 3,
dadurch **gekennzeichnet,** daß
sie ein Maleinanhydrid/Methylvinylether-Copolymer enthält, das mit 1 bis 5 Mol%, bezogen auf den Methylvinylether, 1,9-Decadien oder 1,7-Octadien vernetzt ist.

5. Zusammensetzung gemäß jedem der Ansprüche 1 bis 4,
dadurch **gekennzeichnet,** daß
die nicht-ionischen oberflächenaktiven Mittel der Familie der Alkylpolyglycoside die folgende Formel aufweisen:
R(C₆H₁₀O₅)ₓ-H (II)
mit der folgenden Strukturformel: 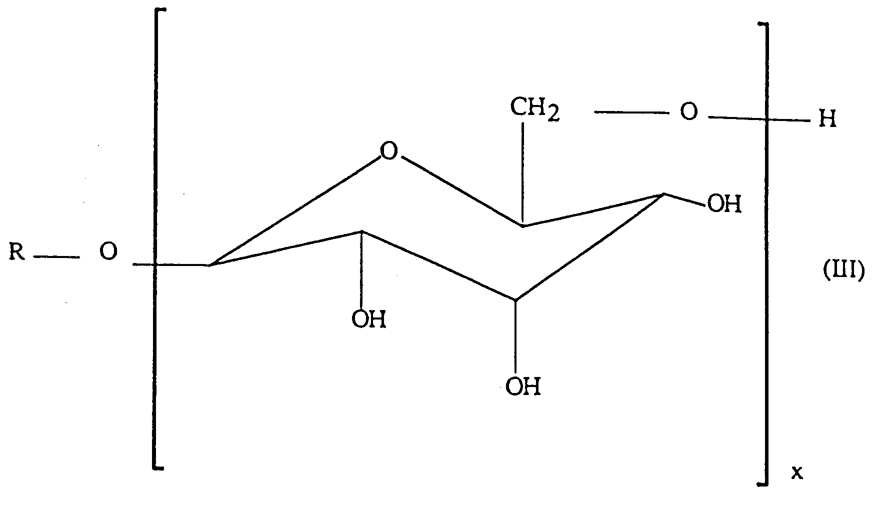 worin gilt:
R bedeutet einen geradkettigen oder verzweigten C₈₋₂₄-Alkyl- oder -Alkenylrest oder eine Mischung dieser Reste;
x bedeutet eine Zahl von 1 bis 15.

6. Zusammensetzung gemäß einem der Ansprüche 1 bis 5,
dadurch **gekennzeichnet,** daß
die nicht-ionischen oberflächenaktiven Mittel der Polyglycerylverbindungen aus den folgenden Polhydroxypropylethern ausgewählt sind:
(A) aus Verbindungen der Formel (IV):
RO(C₃H₅(OH))ₙ-H (IV)
worin die Gruppierung (C₃H₅(OH)), jeweils zusammen oder alleine, die folgenden Strukturen darstellt:
⁅CH₂CHOH-CH₂-O⁆ (IVa)
, und 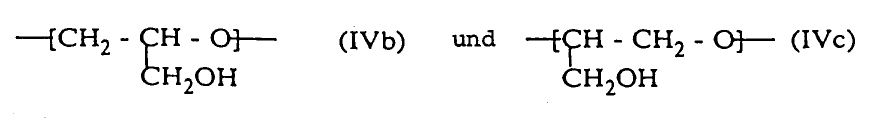 und worin R und n die folgenden Bedeutungen haben:
a) R stellt einen C₁₀₋₁₄-Alkylrest oder eine Mischung dieser Reste dar, und n ist eine ganze Zahl oder Dezimalzahl von 2 bis 10 und vorzugsweise von 3 bis 6;
b) R stellt einen Rest dar:
R₂CONHCH₂-CH₂OCH₂-CH₂- (V)
worin R₂ einen C₁₁₋₁₇-Alkyl- und/oder -Alkenylrest oder eine Mischung dieser Reste bedeutet, und n bedeutet eine ganze Zahl oder Dezimalzahl von 1 bis 5 und vorzugsweise von 1,5 bis 4;
c) R stellt einen Rest dar:
R₃-CHOH-CH₂- (VI)
worin R₃ einen aliphatischen, cycloaliphatischen oder arylaliphatischen C₇₋₂₁-Rest und deren Mischungen bedeutet, wobei die aliphatischen Ketten, die insbesondere Alkylketten bedeuten, 1 bis 6 Ether-, Thioether- und/oder Hydroxymethylen-Gruppierungen aufweisen können, und n bedeutet eine ganze Zahl oder Dezimalzahl von 1 bis 10;
(B) aus Verbindungen, die durch unter saurer Katalyse durchgeführte Kondensation von 2 bis 10 und vorzugsweise von 2,5 bis 6 Mol Glycidol pro Mol Alkohol und α-Diol, welche 10 bis 14 Kohlenstoffatome enthalten, bei einer Temperatur von 50 bis 120°C hergestellt sind, wobei das Glycidol langsam zum Alkohol oder α-Diol gegeben wird;
(C) aus Polyhydroxypropylether-Verbindungen, die durch Polyaddition von Glycerylmonochlorhydrin an eine polyhydroxylierte organische Verbindung in Gegenwart einer starken Base unter schrittweiser destillativer Eliminierung von Wasser hergestellt sind.

7. Zusammensetzung gemäß Anspruch 6,
dadurch **gekennzeichnet,** daß
die nicht-ionischen oberflächenaktiven Mittel der Polyglycerylverbindungen aus den folgenden Polyhydroxypropylethern ausgewählt sind:
(α) 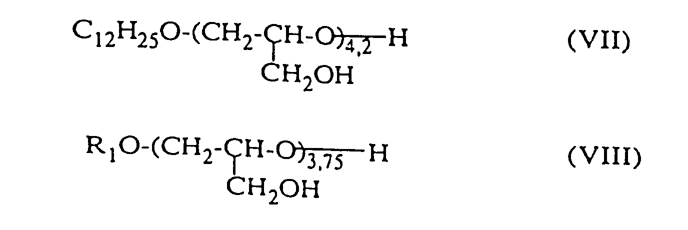 worin R₁ eine Mischung aus C₁₀H₂₁- und C₁₂H₂₅-Alkylresten bedeutet;
(β) aus Verbindungen, die durch unter alkalischer Katalyse durchgeführte Kondensation von 3,5 Mol Glydidol mit einem 12 Kohlenstoffatome aufweisenden α-Diol hergestellt sind,;
(γ) aus Verbindungen der Formel:
R₂-CONH-CH₂-CH₂-O-CH₂-CH₂-O-(CH₂-CHOH-CH₂-O)̵_{3,5}H (IX)
worin R₂ eine Mischung aus Resten bedeutet, welche die folgenden Alkyl- und Alkenylreste umfassen: C₁₁H₂₃, C₁₃H₂₇, aus Fettsäuren von Kopra abgeleitete Reste und den von Ölsäure abgeleiteten Rest;
(δ) aus Verbindungen, die durch Kondensation von 3,5 Mol Glycidol an eine Mischung aus C₁₁₋₁₄-α-Diolen hergestellt sind.

8. Zusammensetzung gemäß einem der Ansprüche 1 bis 7,
dadurch **gekennzeichnet,** daß
sie 0,2 bis 5 und vorzugsweise 0,3 bis 3 Gew.% vernetztes Copolymer enthält, bezogen auf das Gesamtgewicht der Zusammensetzung.

9. Zusammensetzung gemäß einem der Ansprüche 1 bis 8,
dadurch **gekennzeichnet,** daß
sie 0,1 bis 5 Gew.%, bezogen auf das Gesamtgewicht der Zusammensetzung, an nicht-ionischen oderflächenaktiven Mitteln der Familie der Alkylpolyglycoside und/oder der Polyglycerylverbindungen enthält.

10. Zusammensetzung gemäß einem der Ansprüche 1 bis 8,
dadurch **gekennzeichnet,** daß
sie 5 bis 30 und vorzugsweise 5 bis 20 Gew.%, bezogen auf das Gesamtgewicht der Zusammensetzung, an nicht-ionischen oberflächenaktiven Mitteln der Familie der Alkylpolyglycoside und/oder der Polyglycerylverbindungen enthält.

11. Zusammensetzung gemäß einem der Ansprüche 1 bis 10,
dadurch **gekennzeichnet,** daß
das kosmetisch geeignete Medium ein wässriges Medium ist, das alleiniglich aus Wasser oder aus einer Mischung aus Wasser und einen kosmetisch geeigneten Lösungsmittel zusammengesetzt ist.

12. Zusammensetzung gemäß einem der Ansprüche 1 bis 11,
dadurch **gekennzeichnet,** daß
sie in Form einer mehr oder weniger verdickten Flüssigkeit, eines Gels, einer Emulsion, einer hydrolakoholischen Lotion, einer Dispersion oder eines Aerosol-Schaums vorliegt.

13. Zusammensetzung gemäß einem der Ansprüche 1 bis 12,
dadurch **gekennzeichnet**, daß
sie anionische, kationische, amphotere oder zwitterionische oberflächenaktive Mittel, weitere nicht-ionische oberflächenaktive Mittel, anionische, kationische, nichtionische oder amphotere Polymere, Proteine, Kohlenwasserstofföle, Öle, Wachse, Harze oder Gummiprodukte aus Silicon, sauer oder alkalisch stellende Mittel, Konservierungsmittel, Wirkstoffe, weitere Verdickungsmittel, Suspendiermittel, weichmachende Mittel, Sonnenfilterstoffe, Parfüm-Produkte, Biozide, Anitoxidantien, Fluorverbindungen, Pigmente oder weitere gewöhnlich in der Kosmetik eingesetzte Hilfsstoffe enthält.

14. Kosmetische Verwendung der in einem der Ansprüche 1 bis 13 definierten Zusammensetzung zur Behandlung und/oder zum Waschen keratinischer Materien aus Haaren oder der Haut.

15. Verfahren zur kosmetischen Behandlung,
dadurch **gekennzeichnet,** daß
es darauf beruht, daß man auf die Haut oder die Haare eine wirksame Menge einer Zusammensetzung gemäß einem der Ansprüche 1 bis 13 aufbringt.

16. Verfahren zur kosmetischen Behandlung gemäß Anspruch 15,
dadurch **gekennzeichnet,** daß
die Zusammensetzung in einer kosmetisch wirksamen Menge auf feuchte oder trockene Haare aufgebracht wird, um diese zu waschen, und daß man sodann eine Spülung mit Wasser durchführt.
